(19) Europäisches Patentamt / European Patent Office / Office européen des brevets

(11) **EP 4 382 906 A1**

(12) **EUROPEAN PATENT APPLICATION**

(43) Date of publication:
**12.06.2024 Bulletin 2024/24**

(21) Application number: **23213902.2**

(22) Date of filing: **04.12.2023**

(51) International Patent Classification (IPC):
**G01N 33/44** *(2006.01)*    **G01N 1/22** *(2006.01)*
**G01N 30/00** *(2006.01)*    **G01N 30/86** *(2006.01)*

(52) Cooperative Patent Classification (CPC):
**G01N 33/442; G01N 30/00; G01N 30/86;
G01N 30/8606;** G01N 1/2226

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **07.12.2022 IT 202200025134**

(71) Applicants:
• **SERMAG S.r.l.**
  **20124 Milano (IT)**
• **Mottin S.r.l.**
  **21012 Cassano Magnago (VA) (IT)**

(72) Inventors:
• **GIANOTTI, Valentina**
  **I-15033 CASALE MONFERRATO (Alessandria)
  (IT)**
• **LAUS, Michele**
  **I-15033 CASALE MONFERRATO (Alessandria)
  (IT)**
• **MOTTIN, Mauro**
  **I-21012 CASSANO MAGNAGO (Varese) (IT)**
• **ROSMINO, Andrea**
  **I-16146 GENOVA (IT)**

(74) Representative: **Aseglio-Gianinet, Romina et al
Jacobacci & Partners S.p.A.
Corso Emilia, 8
10152 Torino (IT)**

(54) **METHOD FOR EVALUATING THE PROFILE OF VOLATILE AND ODOROUS SUBSTANCES IN A MATERIAL**

(57) The method comprises a simple headspace sampling and GC-MS analysis of samples of the test material. A statistically significant comparison between samples is obtained by calibration with reference samples that ensure reproducibility of the results against a possible change in sensitivity of the gas chromatographic instrument. The method is quick to perform as the data obtained are analysed by integrating areas of a chromatogram without identifying and assigning each chromatographic peak individually.

FIG. 1

EP 4 382 906 A1

**Description**

The present invention refers to a method for evaluating the profile of volatile and odorous substances in a material, such as polymeric material, in particular from the recycling of pre-consumer and post-consumer plastic waste.

Such a profile can typically be used to evaluate the tendency of the material to emit odours, which is a critical parameter for the suitability of this material for most uses.

The evaluation of the profile of the volatile component is not covered by specific regulations. However, a number of methods are known in the literature which comprise several steps of extraction, clean-up, and chromatography analysis.

The evaluation of the odorousness of a material, instead, is covered by regulations UNI EN 13725:2022, VDA 278, and VDA 270, the latter being the simplest.

In particular, the VDA 270 standard requires the use of a panel of at least three trained persons who evaluate samples subjected to storage and heat treatment.

However, neither the VDA 270 standard nor the VDA 278 standard can be used during a production process, because the frequency of analysis and the timing cannot be adapted to the needs of such a process that requires continuous checks and evaluations.

Therefore, it is an object of the present invention to overcome the above-mentioned issues of the prior art.

According to the invention, this object is achieved by means of a method for evaluating the profile of volatile and odorous substances in a material, comprising the steps of:

- placing into at least one vial a sample of the material to be evaluated and into a further vial a calibration sample, wherein said vials have a headspace,
- sealing said vials and heating them for a predetermined period of time,
- taking an amount of gas from the headspace of each vial and successively injecting said amounts of gas into a gas chromatograph coupled to a mass spectrometer, so as to carry out a gas chromatography-mass spectrometry (GC-MS) analysis for both the sample of material to be evaluated and the calibration sample and obtain the respective gas-chromatograms,

- dividing said gas-chromatograms into a same number of sequential zones which is at least 3, and integrating the total area of each zone,
- for each zone, calculating the value

$$AnWS = (AnW / AStdn),$$

wherein

AnW is the total area of the gas-chromatogram zone of the material sample to be evaluated divided by the weight of the material sample to be evaluated, and
AStdn is the total area of the corresponding zone of the gas-chromatogram of the calibration sample divided by the weight of the calibration material sample,
wherein the individual AnWS values and their sum ATOTWS constitute parameters identifying said profile of volatile and odorous substances.

Preferred embodiments of the method of the invention are described in the claims dependent on claim 1 below.

Advantageously, the method of the invention comprises a simple headspace sampling and GC-MS analysis of the test sample. A statistically significant comparison between different samples is obtained by calibration with standard reference samples that ensure reproducibility of the data against a possible change in sensitivity of the gas chromatograph.

Preferably, the sample of the material to be evaluated is supplied in the form of pellets, granules, flakes, or powders, which may be obtained by conventional grinding procedures, and in particular with a particle size not greater than 7 mm.

One advantage of the method of the invention is the extremely short implementation time, due to the fact that the data can be obtained by simply integrating areas of the chromatogram, without having to identify and assign each chromatographic peak individually. In particular, the method of the invention may involve carrying out the test with a duration of approximately 120 minutes. For sequential testing, synchronizing the headspace heating time with the chromatographic run allows the profile of the volatile and odorous substances to be obtained in half the time.

The method of the invention has the additional advantage of not requiring any particular pretreatment of the sample, or the replication of measurements, or the use of non-conventional equipment and instruments.

The method of the invention is applicable for timely, rapid and continuous evaluations during an industrial production process. More generally, the method of the invention can be used in all cases requiring rapid evaluations and repeated tests in a short time, which would be impossible to carry out in a similar time period with conventional methods.

The method of the present invention is economically advantageous, since the related costs are much lower than those of similar tests carried out in external laboratories according to the VDA 270 and VDA 278 methods or the UNI EN 13725:2022 standard.

A further advantage of the method of the present invention is its suitability for the continuous control of industrial processes that are subject to great variability in the quality of the input material, for example processes for recycling polymeric waste, which - despite this variability - must nevertheless ensure high homogeneity and stability of the concentration profile of the volatile substances in the finished product.

The method of the present invention is applicable to verify the efficiency of any process aimed at reducing the content of volatile substances in a polymeric material. By analysing the material before and after carrying out the process for reducing the volatile substances, the results thus obtained can be compared to show the actual degree of reduction achieved in the content of volatile substances. In turn, this degree of reduction can be correlated with the reduction in the odour of the processed material, if the volatile substances are odoriferous.

The method of the invention is also applicable to non-polymeric materials, provided that they are compatible with each of its steps.

Further advantages and features of the present invention will be apparent from the detailed description which follows, given by way of non-limiting example with reference to the appended drawings, in which:

> Figure 1 is a diagram of the zoning of a chromatogram used in a method according to the invention and of the acquisition of the related area value,
> Figure 2 is a diagram for the placement of a respective standard substance in each zone of a chromatogram used in a method according to the invention,
> Figure 3 is a chromatogram of a sample of material as such, obtained in an implementation of the method according to the invention, and
> Figure 4 is a chromatogram of a sample of the same material after a deodorization treatment, obtained in an implementation of the method according to the invention.

A weighed amount of pellets, advantageously between 100 and 500 mg, of the material to be evaluated with regard to the profile of volatile substances is introduced into a pre-weighed glass vial having a headspace of approximately 10 ml. The vial is immediately closed with a PTFE/silicone pierceable cap and stored in the dark at room temperature.

In order to have a sufficient number of samples to obtain true replicas, several hermetically sealed vials can be prepared at the same time for separate analysis, if necessary.

Each vial is then advantageously heated to a temperature at least 10 °C below the melting temperature of the test material for a period of time in the range between 30 and 90 minutes. For example, if the material to be evaluated is a polyolefin, the optimal temperature for this heating step is between 120 and 140°C and the related time is 1 hour.

Subsequently, an amount of gas between 3 and 10 $\mu$l is taken from the headspace of each vial using a gastight glass syringe either manually or via an autosampler.

In parallel, a calibration sample is also prepared by placing an inert material, in the same proportions as the sample to be evaluated, on the bottom of a similar headspace-equipped vial, to which a mixture of standard substances is added. The selection of the standard substances must only meet the requirement of having one molecule with a retention time for each zone into which the chromatogram obtained from a GC-MS analysis will be divided, as will be further detailed. The optimal concentration to which the standard substances shall be added is between 0.1 and 5 mg/l and the selection thereof falls within the skills of a person of average skill in gas chromatography.

The amounts of gas taken for each sample of the material to be evaluated and the calibration material are then separately analysed in the same way by GC-MS using a conventional apparatus consisting of a gas chromatograph coupled to a mass spectrometer.

Following the GC-MS analysis, a respective chromatogram is obtained for the sample to be evaluated and the calibration sample, which exhibits a plurality of peaks each corresponding to a more or less volatile substance occurring in the sample.

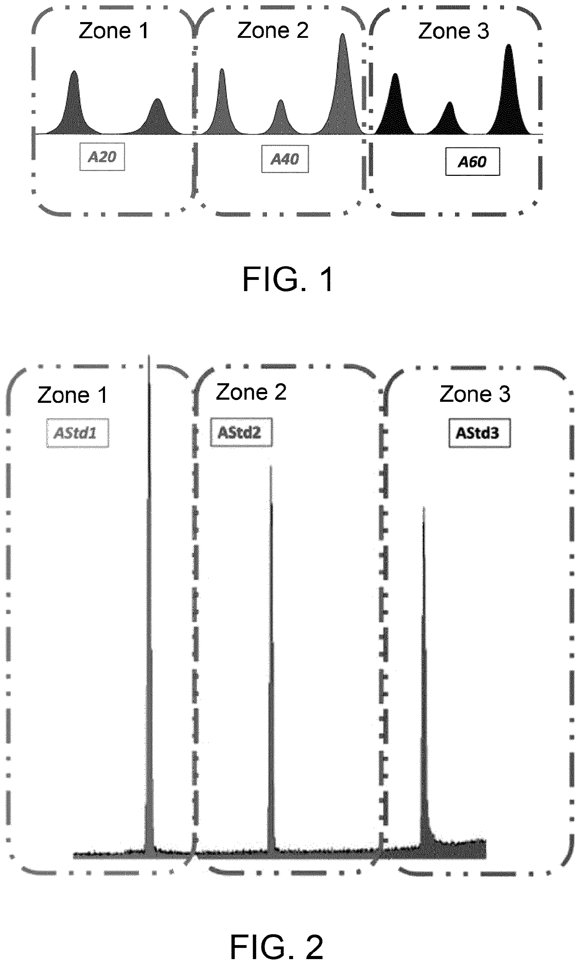

Each chromatogram thus obtained is divided into *n* zones, with n advantageously between 3 and 5 and, for each zone, the total area (i.e., the area under the sequence of peaks) is integrated, as schematically shown in Figure 1 with reference to a division of a chromatogram into three zones. This can be done manually in full scan mode. Figure 2, on the other hand, shows the positioning of a respective standard substance that has been carried out in each of the three zones of the chromatogram of the calibration sample.

The selection of a number of zones equal to 3 is the best compromise between, on the one hand, the quickness and simplicity of the method and, on the other hand, the quality of the information that can be obtained from it.

Typically, the most volatile compounds correspond to the peaks in the first zone of the chromatogram, while the low slope of the temperature gradient also allows the less volatile compounds to be resolved. In greater detail, the above three zones have the following characteristics with reference to an overall analysis time of 60 minutes.

Zone 1 corresponds to the area between the beginning of the chromatogram and 20.0 minutes and allows the assessment of the volatile component It is referred to as *A20.*

Zone 2 corresponds to the area between 20.01 minutes and 40.0 minutes and allows the assessment of the semi-volatile component. It is referred to as *A40.*

Zone 3 corresponds to the area between 40.01 minutes and 60.0 minutes and allows the assessment of the low volatility component. It is referred to as *A60.*

Then, the values *A20, A40 and A60* are divided by the sample weight (*W*) to obtain the values *A20W*, *A40W and A60W.* Finally, these values are scaled by dividing them by the area of the corresponding zone of the chromatogram of the calibration sample, also divided by the weight of the calibration sample (*Astd1, Astd2, Astd3).* The scaled values are referred to as *A20WS, A40WS and A60WS,* respectively.

If the choice is made to divide the chromatogram into more than 3 zones, more detailed information can be obtained, but it is necessary to identify a respective standard substance with a retention time included in each of the different zones.

In general, if the chromatogram is divided into a different number of zones, the above values are generically referred to as *AnW and AnWS,* where:

$$AnW = An \, / \, W \qquad\qquad (1)$$

$$AnWS = (AnW \, / \, AStdn) \qquad\qquad (2)$$

where *n* represents the cumulative number of minutes in each zone.

The sum of all the *AnWS* values, i.e., the total value referred to as *ATOTWS,* is a synthetic parameter identifying the evaluated material and allows for a rapid comparison of different materials with reference to the total volatile substances present therein.

The *AnWS* values, on the other hand, are parameters identifying a given material with reference to the volatile substances present therein and having a volatility within a given range, and therefore allow a comparison restricted to this range.

In general, the above AnWS and ATOTWS values constitute a semi-quantitative and statistically significant result, which allows the comparison of different samples.

A specific non-limiting embodiment of an evaluation method according to the invention is now described.

### *Example*

The material under evaluation is a secondary raw material consisting of polypropylene pellets from the post-consumer recycling chain, and therefore potentially containing volatile foreign substances, in particular susceptible to generating odours. This material is evaluated as such ($C_{as}$) and after a deodorization treatment ($C_{treat}$) of the kind described in Italian patent application No 102021000028754 filed on 12/11/2021.

Approximately 500 mg of pellets of each material sample are placed into a respective glass vial having a headspace of 10 ml, which is then immediately closed with a PTFE/silicone pierceable cap and stored in the dark at room temperature.

Each vial is heated to a temperature of 130°C for 1 hour. An amount of 5 $\mu$l of gas is then taken from the headspace by means of a gas-tight glass syringe and subjected to GC-MS analysis using a Phenomenex ZB-Wax MS capillary column (30m, 0.25 mm i.d., 0.25 $\mu$m thick), an injector temperature of 250 ∘C, operating in splitless mode, and helium as the carrier gas at 1.0 mL min-1. The MS transfer line and oven temperatures are held at temperatures of 300 and 150 ∘C, respectively. The MS signal acquired in EI+ mode with ionization energy of 70.0 eV and ion source temperature of 290 ∘C is used. The acquisition is performed in full scan mode in the range of 35-500 m/z. The column heating program consists of the slope steps shown in Table 1 below and has a total duration of 60 minutes. A solvent delay of 5 minutes is set.

Table 1

| GC oven temperature program | | | |
|---|---|---|---|
| | Heating rate (°C/min) | T | Isocratic time |
| | | (°C) | (min) |
| Initial | | 45 | 0.10 |
| Ramp 1 | 5.0 | 90 | 0.10 |
| Ramp 2 | 4.0 | 150 | 10.00 |
| Ramp 3 | 5.0 | 230 | 10.00 |

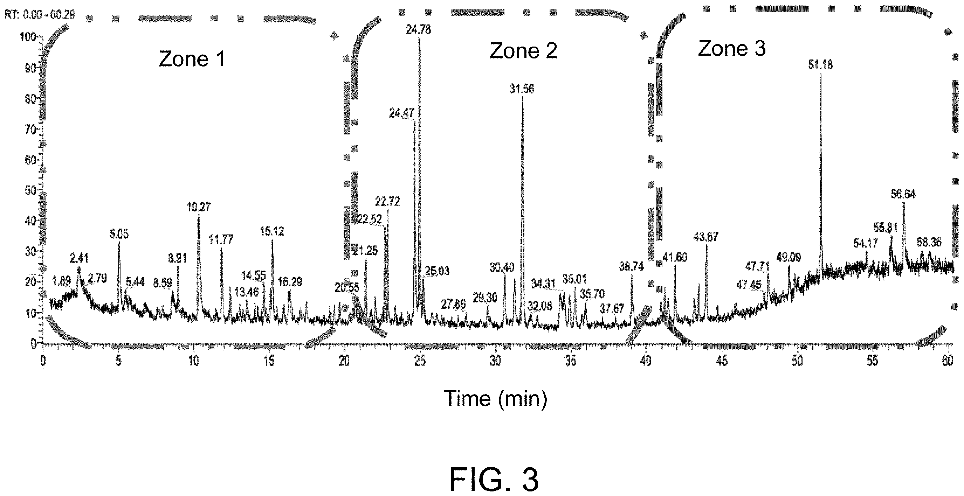

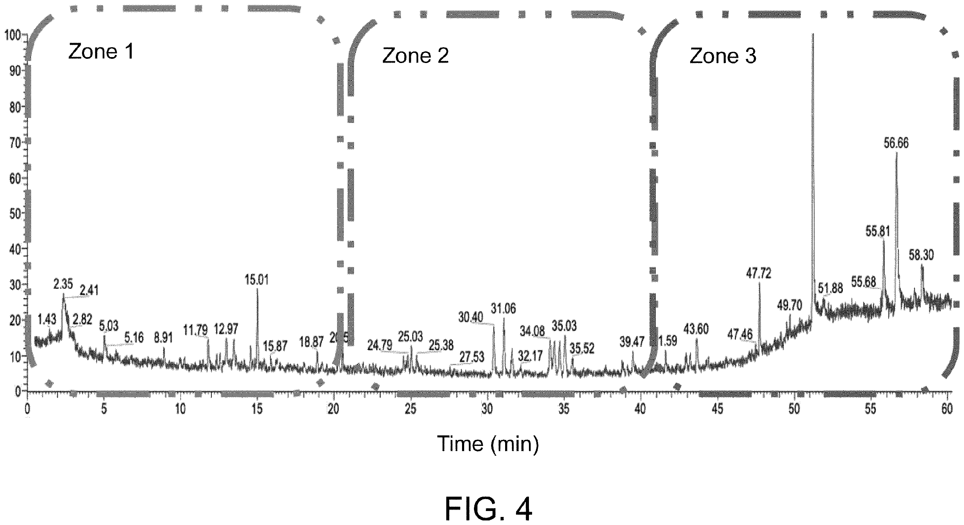

Figures 3 and 4 show the chromatograms obtained for the sample as such and after treatment, respectively, which have been divided into three zones.

The integration of the area under the respective gas chromatogram in each zone is carried out using the native software of the gas chromatograph.

The An, AnW, AnWS, and Astd values relating to the sample both as such and after treatment, as well as the total ATOTWS value, are then calculated for each of the three zones. These values are given in Table 2 below.

Table 2

| | Cas | Ctreat |
|---|---|---|
| sample weight (g) | 0.5730 | 0.7483 |
| Astd1 | 12,399,215 | 20,435,937 |
| Astd2 | 19,336,248 | 30,752,772 |
| Astd3 | 12,581,325 | 25,941,191 |
| std weight (mg) | 1 | 1 |
| A20 | 107,514,025 | 10,437,309 |
| A40 | 152,436,799 | 96,450,871 |
| A60 | 191,813,174 | 26,946,353 |
| A20W | 187,633,551 | 13,948,028 |
| A40W | 266,032,808 | 128,893,320 |
| A60W | 334,752,485 | 36,010,094 |
| A20WS | 9 | 1 |
| A40WS | 8 | 3 |
| A60WS | 15 | 1 |
| ATOTWS | 32 | 5 |

As shown in Table 2, the ATOTWS values clearly indicate that the overall volatile substances have decreased considerably as a result of the treatment and, where appropriate, allow the parameters of the treatment to be corrected so as to promptly increase the effectiveness thereof.

Of course, without prejudice to the invention principle, the implementation details and embodiments may vary widely from what is described purely by way of example, without however departing from the scope of the invention as defined in the attached claims.

**Claims**

1.   A method for evaluating the profile of volatile and odorous substances in a material, comprising the steps of:

- placing into at least one vial a sample of the material to be evaluated and into a further vial a calibration sample, wherein said vials have a headspace,

- sealing said vials and heating them for a predetermined period of time,
- taking an amount of gas from the headspace of each vial and successively injecting said amounts of gas into a gas chromatograph coupled to a mass spectrometer, so as to carry out a gas chromatography-mass spectrometry (GC-MS) analysis for both the sample of material to be evaluated and the calibration sample and obtain the respective gas-chromatograms,
- dividing said gas-chromatograms into a same number of sequential zones which is at least 3, and integrating the total area of each zone,
- for each zone, calculating the value

$$AnWS = (AnW / AStdn),$$

wherein

AnW is the total area of the gas-chromatogram zone of the material sample to be evaluated divided by the weight of the material sample to be evaluated, and
AStdn is the total area of the corresponding zone of the gas-chromatogram of the calibration sample divided by the weight of the calibration material sample,
wherein the individual AnWS values and their sum ATOTWS constitute parameters identifying said profile of volatile and odorous substances.

2. The method according to claim 1, wherein a respective sample of the material to be evaluated is introduced into a plurality of vials and an arithmetic mean of the values obtained for each sample of the material to be evaluated, respectively, constitutes the AnWS value of the sample of the material to be evaluated.

3. The method according to any one of the preceding claims, wherein the samples of the material to be evaluated and calibration material, respectively, are in the form of pellets, granules, flakes, or powders.

4. The method according to claim 3, wherein said pellets, granules, flakes, or powders have a particle size not greater than 7 mm.

5. The method according to any one of the preceding claims, wherein said material to be evaluated is a polymeric material.

6. The method according to any one of the preceding claims, wherein each of said vials has a headspace of approximately 10 ml.

7. The method according to any one of the preceding claims, wherein each gas-chromatogram is divided into a number of sequential zones comprised between 3 and 5.

8. The method according to any one of the preceding claims, wherein the duration of the GC-MS analysis for each sample is in the range between 30 and 90 minutes.

9. The method according to any one of the preceding claims, wherein said amount of gas taken from each vial is in the range between 3 and 10 $\mu$l.

10. The method according to any one of the preceding claims, wherein said vials are heated to a temperature at least 10 °C below the melting temperature of the material to be evaluated for the predetermined period of time which is in the range between 30 and 90 minutes.

11. The method according to any one of the preceding claims, wherein the step of heating the vial of one sample takes place at the same time as the step of GC-MS analysis of another sample.

FIG. 1

FIG. 2

FIG. 3

FIG. 4

EP 4 382 906 A1

Europäisches
Patentamt

European
Patent Office

Office européen
des brevets

# EUROPEAN SEARCH REPORT

**Application Number**

EP 23 21 3902

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TAVARES DIVA S. ET AL: "Determination and Profiling of Human Skin Odors Using Hair Samples", MOLECULES, vol. 24, no. 16, 15 August 2019 (2019-08-15), page 2964, XP093052549, DOI: 10.3390/molecules24162964 | 1,2,6-11 | INV. G01N33/44 G01N1/22 G01N30/00 G01N30/86 |
| Y | * page 3, paragraph 2.1(b) * <br> * page 5, paragraph 2.3(b) - paragraph 2.3(c) * <br><br>----- | 3-5 | |
| Y | PAJARO-CASTRO NERLIS ET AL: "Identification of volatile organic compounds (VOCs) in plastic products using gas chromatography and mass spectrometry (GC/MS)", AMBIENTE E AGUA - AN INTERDISCIPLINARY JOURNAL OF APPLIED SCIENCE, vol. 9, no. 4, 1 December 2014 (2014-12-01), pages 1-11, XP093052206, DOI: 10.4136/ambi-agua.1435 * page 612, line 1, paragraph 2 - line 25 * <br><br>----- | 3-5 | TECHNICAL FIELDS SEARCHED (IPC) <br><br> G01N |
| A | Jacq Karine ET AL: "Analysis of Volatile Organic Compounds in Water Using Static Headspace-GC/MS", Application Note, 9 December 2008 (2008-12-09), pages 1-16, XP093052547, Retrieved from the Internet: URL:https://www.agilent.com/cs/library/app lications/5990-3285EN.pdf [retrieved on 2023-06-07] * the whole document * <br><br>----- <br><br>-/-- | 1-11 | |

The present search report has been drawn up for all claims

| Place of search | Date of completion of the search | Examiner |
|---|---|---|
| The Hague | 11 March 2024 | Joyce, David |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

page 1 of 2

Europäisches
Patentamt

European
Patent Office

Office européen
des brevets

# EUROPEAN SEARCH REPORT

**Application Number**

**EP 23 21 3902**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CABANES A. ET AL: "A review on VOCs from recycled plastics", SUSTAINABLE MATERIALS AND TECHNOLOGIES, vol. 25, 1 September 2020 (2020-09-01), page e00179, XP093052200, ISSN: 2214-9937, DOI: 10.1016/j.susmat.2020.e00179 * the whole document * | 1-11 | |
| A | MEGGIE HAKIM ET AL: "Volatile Organic Compounds of Lung Cancer and Possible Biochemical Pathways", CHEMICAL REVIEWS, vol. 112, no. 11, 14 November 2012 (2012-11-14), pages 5949-5966, XP055153082, ISSN: 0009-2665, DOI: 10.1021/cr300174a * the whole document * | 1-11 | |

| | | | TECHNICAL FIELDS SEARCHED (IPC) |
|---|---|---|---|

The present search report has been drawn up for all claims

| Place of search | Date of completion of the search | Examiner |
|---|---|---|
| The Hague | 11 March 2024 | Joyce, David |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**page 2 of 2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IT 102021000028754